(19) Europäisches Patentamt
European Patent Office
Office européen des brevets

(11) EP 4 317 359 A1

(12) EUROPEAN PATENT APPLICATION
published in accordance with Art. 153(4) EPC

(43) Date of publication:
**07.02.2024 Bulletin 2024/06**

(21) Application number: **21933201.2**

(22) Date of filing: **30.09.2021**

(51) International Patent Classification (IPC):
***C09K 9/02*** *(2006.01)* ***G02C 7/10*** *(2006.01)*
***C07D 311/78*** *(2006.01)* ***C08F 2/44*** *(2006.01)*

(52) Cooperative Patent Classification (CPC):
**C07D 311/78; C08F 2/44; C09K 9/02; G02C 7/10**

(86) International application number:
**PCT/JP2021/036066**

(87) International publication number:
**WO 2022/201602 (29.09.2022 Gazette 2022/39)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **26.03.2021 JP 2021054000**

(71) Applicant: **Hoya Lens Thailand Ltd.**
**Pathumthani 12130 (TH)**

(72) Inventors:
- **SHIMADA, Takuya**
  **Tokyo 160-8347 (JP)**
- **NUSHI, Yusuke**
  **Tokyo 160-8347 (JP)**
- **YAMASHITA, Teruo**
  **Tokyo 160-8347 (JP)**

(74) Representative: **Hoffmann Eitle**
**Patent- und Rechtsanwälte PartmbB**
**Arabellastraße 30**
**81925 München (DE)**

# (54) INDENO-FUSED NAPHTHOPYRAN COMPOUND PARTICLES, METHOD FOR PRODUCING POLYMERIZABLE COMPOSITION, AND METHOD FOR PRODUCING PHOTOCHROMIC ARTICLE

(57) There are provided indeno-fused naphthopyran compound particles having a degree of crystallization of 10.00% or less and a maximum primary particle size of 200 um or less.



Processed by Luminess, 75001 PARIS (FR)

## Description

[Technical Field]

The present invention relates to indeno-fused naphthopyran compound particles, a method of producing a polymerizable composition and a method of producing a photochromic article.

[Background Art]

PTL 1 discloses an indeno-fused naphthopyran compound.

[Citation List]

[Patent Literature]

[PTL 1] U.S. Patent No. 5645767

[Summary of Invention]

[Technical Problem]

A photochromic compound is a compound having a property of developing a color under light emission of light in a wavelength range having photoresponsivity and fading without light emission (photochromism) (for example, refer to PTL 1). In recent years, the usefulness of indeno-fused naphthopyran compounds as photochromic compounds has been focused on.

Examples of articles to which photochromism is imparted from photochromic compounds (photochromic articles) include optical articles such as spectacle lenses. Examples of methods of imparting photochromism to photochromic articles include a method of providing a photochromic layer on a substrate using a polymerizable composition containing a photochromic compound and a polymerizable compound and a method of producing a substrate using a polymerizable composition containing a photochromic compound and a polymerizable compound.

In a polymerizable composition containing a photochromic compound and a polymerizable compound, high solubility of the photochromic compound in the polymerizable composition is desired. If the solubility of the photochromic compound can be improved, it is possible to reduce the dispersion particle size of the photochromic compound in the polymerizable composition. Since reducing the dispersion particle size of the photochromic compound can contribute to improving optical homogeneity of the layer and/or the substrate formed using the polymerizable composition, this is desirable in order to improve the appearance quality of photochromic articles.

In view of the above, an object of one aspect of the present invention is to provide a means that can improve the solubility of an indeno-fused naphthopyran compound in a polymerizable composition containing an indeno-fused naphthopyran compound and a polymerizable compound.

[Solution to Problem]

One aspect of the present invention relates to indeno-fused naphthopyran compound particles having a degree of crystallization of 10.00% or less and a maximum primary particle size of 200 um or less (hereinafter simply referred to as "particles").

[Advantageous Effects of Invention]

According to indeno-fused naphthopyran compound particles of one aspect of the present invention, it is possible to provide a polymerizable composition containing an indeno-fused naphthopyran compound and a polymerizable compound, wherein the indeno-fused naphthopyran compound has high solubility in the composition.

[Description of Embodiments]

[Indeno-fused Naphthopyran Compound Particles]

One aspect of the present invention relates to indeno-fused naphthopyran compound particles. The degree of crystallization of the particles is 10.00% or less and the maximum primary particle size is 200 um or less.

The inventors thought that indeno-fused naphthopyran compound particles having a degree of crystallization of 10.00% or less are amorphous or particles in a mixed form of amorphous and crystalline. Here, the term "crystalline" is used to include both single crystals and polycrystals in which a large number of crystal grains are aggregated. The term "crystalline" refers to a crystallographic single crystal or polycrystal state such as peaks observed in X-ray diffraction and/or electron beam diffraction. The term "amorphous" refers to a crystallographically amorphous state such as halos observed in X-ray diffraction and/or electron beam diffraction. A mixture of amorphous and crystalline is in the form in which amorphous and crystalline are mixed crystallographically such as peaks and halos observed in X-ray diffraction and/or electron beam diffraction. The inventors speculate that the indeno-fused naphthopyran compound particles that are particles present in such a form and have a maximum primary particle size of 200 um or less can exhibit high solubility because they are easily dispersed in the form of fine particles in a liquid during a mixing process when the polymerizable composition is prepared. In addition, in one aspect, the particles can exhibit high solubility with respect to a polymerizable compound. One means for improving the solubility of the photochromic compound in the polymerizable composition containing a photochromic compound and a polymerizable compound is, for example, use of a solvent. On the other hand, if the solubility of the photochromic compound in the polymerizable compound can be improved, it is possible to reduce the amount of the solvent used for preparing the polymerizable composition. It is desirable to be able to reduce the amount of the solvent used because, for example, when a photochromic layer is provided on a substrate using a polymerizable composition containing a photochromic compound and a polymerizable compound, it is possible to minimize deterioration of the substrate due to the solvent contained in the polymerizable composition.

Hereinafter, the particles will be described in more detail.

The particles are indeno-fused naphthopyran compound particles. The indeno-fused naphthopyran compound is a compound containing an indeno-fused naphthopyran which may have a substituent in its structure. The indeno-fused naphthopyran has a structure in which an indeno group is fused to a naphthopyran. Examples of structures of the indeno-fused naphthopyran which may have a substituent include the structure of the following Formula A. In addition, the particles may be so-called monomers containing only one indeno-fused naphthopyran which may have a substituent or so-called multimers containing two or more indeno-fused naphthopyrans which may have a substituent. A plurality of indeno-fused naphthopyrans contained in the multimer each may have the same or different substituents.

[C1]

(Formula A)

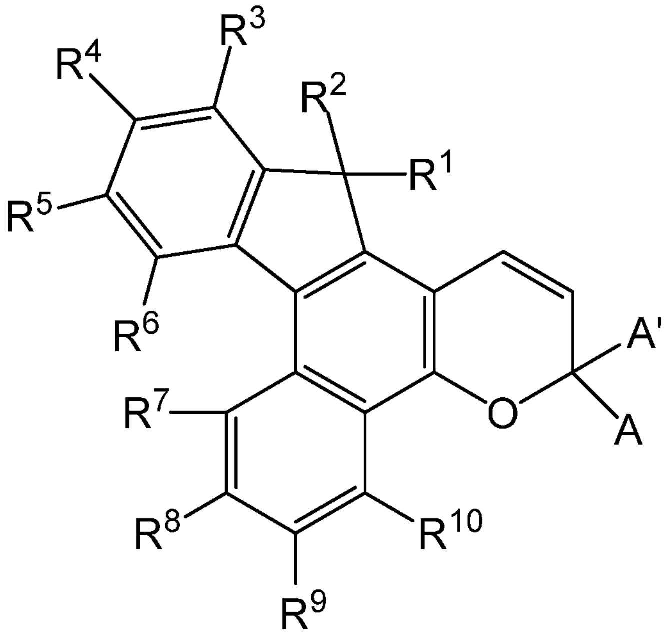

(In Formula A, $R^1$ to $R^{10}$, A and A' each independently represent a hydrogen atom or a substituent.)

As an example, a photochromic compound undergoes structural conversion into a colored component through an excited state when it receives light such as sunlight. The structure after structural conversion via light emission may be called a "colored component." On the other hand, the structure before light emission may be called a "colorless component." However, regarding the colorless component, the term "colorless" is not limited to being completely colorless,

and includes a case in which the color lighter than that of the colored component. The indeno-fused naphthopyran compound can exhibit properties as a photochromic compound (photochromism). The above description regarding the structure of the indeno-fused naphthopyran compound is for the colorless component. The photochromic compound can exhibit photochromism with respect to, for example, ultraviolet rays.

<Degree of Crystallization>

The degree of crystallization of the particles in the present invention and this specification is a value obtained from the following Formula 1 when the results obtained by powder X-ray diffraction analysis are analyzed by the Hermans method.

$$\text{Formula 1: degree of crystallization } X_c (\%) = (I_c/(I_c+I_a)) \times 100$$

In Formula 1, Ic is the integrated intensity of crystalline scattering, and Ia is the integrated intensity of amorphous scattering. The degree of crystallization can be calculated by a known method such as a method using analysis software bundled in an X-ray diffractometer.

Examples of measurement conditions for powder X-ray diffraction analysis include the following measurement conditions.

Target: Cu, X tube current/voltage: 40 mA/45 kV, scanning range: 2θ=4° to 65°

The degree of crystallization of the particles is 10.00% or less, preferably 9.00% or less, more preferably 8.00% or less, still more preferably 7.00% or less, and yet more preferably 6.00% or less. In addition, the degree of crystallization of the particles may be, for example, 0%, 0% or more, more than 0%, 0.10% or more, 0.50% or more, 1.00% or more, 2.00% or more, 3.00% or more, 4.00% or more or 5.00% or more.

<Maximum Primary Particle Size>

The maximum primary particle size of the particles in the present invention and this specification can be obtained by the following method.

The particles to be measured are observed under a reflection type optical microscope at a magnification of about 100. Particles can generally exist as aggregates (secondary particles or higher order particles). The primary particle size is the size of primary particles that constitute the aggregates. The primary particle size is obtained as the length (that is, the major diameter) of the longest straight line as the straight line connecting two points on the contour of the primary particles. The largest value among the major diameter obtained for five randomly extracted primary particles is the maximum primary particle size. The maximum primary particle size of the particles is 200 um or less, preferably 195 um or less, and more preferably 190 um or less. In addition, the maximum primary particle size may be, for example, 100 um or more, 120 um or more, 140 um or more or 160 um or more, or may be below the values exemplified here.

The degree of crystallization of the indeno-fused naphthopyran compound particles can be controlled by increasing the purity during synthesis of the indeno naphthopyran compound, performing a solvent removal treatment during particle preparation or the like, and for example, if the purity is made higher and a larger amount of the solvent is removed, the value of the degree of crystallization tends to be higher. In addition, the maximum primary particle size can be controlled according to heat treatment conditions during particle preparation and the like.

[Method of Producing Polymerizable Composition]

One aspect of the present invention relates to a method of producing a polymerizable composition including mixing the above particles with one or more polymerizable compounds.

The particles can be dissolved in the polymerizable composition with high solubility. Thereby, it is possible to reduce the dispersion particle size of the indeno-fused naphthopyran compound in the polymerizable composition.

<Particles>

The indeno-fused naphthopyran compound particles used for producing the polymerizable composition are as described above.

The indeno-fused naphthopyran compound can exhibit properties as a photochromic compound (photochromism). In the method of producing a polymerizable composition, in one aspect, only one or two or more of the above particles can be used as the photochromic compound, and in another aspect, one or more photochromic compounds may be used in combination in addition to the above particles. Examples of photochromic compounds that can be used in combination include known compounds exhibiting photochromism. Specific examples of photochromic compounds include compounds having a known framework exhibiting photochromism such as fulgimide compounds, spirooxazine compounds, chromene compounds, and indeno-fused naphthopyran compounds. The content of the photochromic compounds based on a total amount (100 mass%) of all components of the polymerizable composition may be, for example, about 0.1 to 15.0 mass%, and but it is not limited to this range. Here, the total amount of all components above is a total amount of all components excluding a solvent when the solvent is used for preparing the polymerizable composition. The content of the particles based on a total amount (100 mass%) of the photochromic compound used for producing the polymerizable composition is preferably 50 mass% or more, more preferably 60 mass% or more, and still more preferably 70 mass% or more. The content of the particles based on a total amount (100 mass%) of the photochromic compound used for producing the polymerizable composition may be, for example, 100 mass%, 100 mass% or less, 95 mass% or less or 90 mass% or less.

Generally, the photochromic compound tends to have low solubility with respect to the polymerizable compound. In order to improve the solubility of the photochromic compound, a solvent is generally used, and if the amount of the solvent used is larger, the solubility of the photochromic compound in the polymerizable composition containing the photochromic compound and the polymerizable composition can be improved. However, as described above, the solvent may cause deterioration of the substrate. In addition, if a solvent is used when a polymerizable composition containing a photochromic compound and a polymerizable compound is prepared, a heat treatment is generally performed in order to volatilize the solvent component when the prepared polymerizable composition is polymerized and cured. However, depending on the solvent, a high-temperature heat treatment is required for volatilization, and such a high-temperature heat treatment may cause deterioration of the substrate. In addition, poor surface quality may occur when the solvent is volatilized. In consideration of the above points, it is desirable that the amount of the solvent used can be reduced by improving the solubility of the photochromic compound in the polymerizable compound. In this regard, the indeno-fused naphthopyran compound can exhibit high solubility with respect to the polymerizable compound due to its particle form. Therefore, when the above particles are used, it is possible to reduce the amount of the solvent used when the polymerizable composition is prepared, and it is possible to use no solvent. As solvents that can be used, any solvent can be used as long as the polymerization reaction of the polymerizable compound can proceed in the presence of such a solvent. The amount of the solvent used when the polymerizable composition is prepared based on a total amount (100 mass%) of all components (including the solvent when the solvent is used) used when the composition is prepared may be, for example, 10.0 mass% or less, 5.0 mass% or less, 3.0 mass% or less, 1.0 mass% or less, or 0 mass%.

<Polymerizable Compound>

In the method of producing a polymerizable composition, the polymerizable compound mixed with the above particles may be of at least 1 type, 2 types or more, or 3 types or more, and may be, for example, 5 types or less or 4 types or less. In the present invention and this specification, the "polymerizable compound" is a compound having a polymerizable group, and the "polymerizable composition" is a composition containing one or more polymerizable compounds. Specific examples of polymerizable groups include an acryloyl group, a methacryloyl group, an acryloyloxy group, and a methacryloyloxy group, and specific examples of polymerizable compounds include (meth)acrylate. In the present invention and this specification, "(meth)acrylate" refers to both an acrylate and a methacrylate. An "acrylate" is a compound having one or more acryloyl groups in one molecule. A "methacrylate" is a compound having one or more methacryloyl groups in one molecule. The number of functional groups of the (meth)acrylate is the number of groups selected from the group consisting of acryloyl groups and methacryloyl groups contained in one molecule. In the present invention and this specification, a "methacrylate" contains only a methacryloyl group as a (meth)acryloyl group, and something that contains both an acryloyl group and a methacryloyl group as (meth)acryloyl groups is an acrylate. The acryloyl group may be contained in the form of an acryloyloxy group, and the methacryloyl group may be contained in the form of a methacryloyloxy group. The "(meth)acryloyl group" mentioned below refers to both an acryloyl group and a methacryloyl group, and "(meth)acryloyloxy group" refers to both an acryloyloxy group and a methacryloyloxy group. In addition, unless otherwise specified, the groups described may have substituents or may be unsubstituted. If a group has a substituent, examples of substituents include an alkyl group (for example, an alkyl group having 1 to 6 carbon atoms), a hydroxy group, an alkoxy group (for example, an alkoxy group having 1 to 6 carbon atoms), a halogen atom (for example, a fluorine atom, a chlorine atom, and a bromine atom), a cyano group, an amino group, a nitro group, an acyl group, and a carboxyl group. In addition, for a group having a substituent, the "number of carbon atoms" is the number of carbon atoms of a part containing no substituents.

(Component A)

In one aspect, in the method of producing a polymerizable composition, the polymerizable compound mixed with the above particles may contain one or more (meth)acrylates. Among (meth)acrylates, an acyclic methacrylate having a molecular weight of 500 or more may be exemplified as one with respect to which the photochromic compound tends to have low solubility. The "acyclic methacrylate having a molecular weight of 500 or more" is also referred to as a "component A." In the method of producing a polymerizable composition, even when the polymerizable compound to be mixed with the photochromic compound contains the component A, if it is mixed in the form of the particles, it is possible to improve the solubility of the indeno-fused naphthopyran compound in the component A.
In the present invention and this specification, "acyclic" means that a compound does not include a cyclic structure. The acyclic methacrylate is a mono- or higher-functional methacrylate that does not include a cyclic structure.
The component A may be a monofunctional or bi-or higher-functional methacrylate, and is preferably a bifunctional or trifunctional methacrylate, and more preferably a bifunctional methacrylate. Examples of components A include polyalkylene glycol dimethacrylate. The polyalkylene glycol dimethacrylate may be represented by the following Formula 1:

[C2]

(Formula 1)

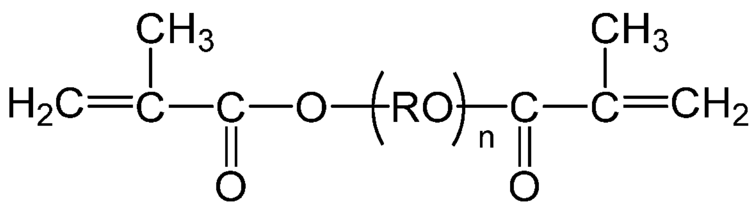

where R represents an alkylene group, and n represents the number of repetitions of alkoxy groups represented by RO, and is 2 or more. Examples of alkylene groups represented by R include an ethylene group, a propylene group, and a tetramethylene group. n is 2 or more, and may be, for example 30 or less, 25 or less or 20 or less. Specific examples of polyalkylene glycol dimethacrylates include polyethylene glycol dimethacrylate, polypropylene glycol dimethacrylate, and polytetramethylene glycol dimethacrylate.
The molecular weight of the component A is 500 or more. It is inferred that the inclusion of an acyclic bifunctional methacrylate (a component A) having a molecular weight of 500 or more together with a component B to be described below can contribute to an ability of a photochromic compound that has received light to develop a color at a high density in a photochromic layer formed from the polymerizable composition containing these components and the photochromic compound. Here, in the present invention and this specification, for the molecular weight of the polymer, a theoretical molecular weight calculated from the structural formula determined by structural analysis of the compound or the raw material preparation ratio during production is used. The molecular weight of the component A is 500 or more, preferably 510 or more, more preferably 520 or more, still more preferably 550 or more, yet more preferably 570 or more, yet more preferably 600 or more, yet more preferably 630 or more, and yet more preferably 650 or more. The molecular weight of the component A is preferably, for example, 2,000 or less, 1,500 or less, 1,200 or less, 1,000 or less, or 800 or less, in order to increase the hardness of the photochromic layer.
The component A may be the most abundant component among the (meth)acrylates used for producing the polymerizable composition, and may be the most abundant component among polymerizable compounds used for producing the polymerizable composition.

(Component B)

In the method of producing a polymerizable composition, examples of polymerizable compounds mixed with the above particles include a bifunctional (meth)acrylate containing a structure selected from the group consisting of cyclic structures and branch structures. The "bifunctional (meth)acrylate containing a structure selected from the group consisting of cyclic structures and branch structures" is also referred to as a "component B."
The component B is a bifunctional (meth)acrylate containing a structure selected from the group consisting of cyclic structures and branch structures. It is inferred that the inclusion of the component A together with the component B can contribute to an ability of a photochromic compound that has received light to develop a color at a high density in

a photochromic layer formed from the polymerizable composition containing these components and the photochromic compound. The component B contains one or more cyclic structures and does not contain a branch structure in one molecule in one aspect, contains one or more branch structures and does not contain a cyclic structure in one molecule in another aspect, and contains one or more cyclic structures and one or more branch structures in one molecule in another aspect. The number of structures selected from the group consisting of cyclic structures and branch structures contained in one molecule is 1 or more, and may be, for example, 1, 2 or 3, and is preferably 1 or 2, and more preferably 1. Regarding the branch structure, when the component B has a methacryloyl group, the branch structure contained in the methacryloyl group is not considered.

In one aspect, the component B containing one or more cyclic structures may be an alicyclic bifunctional (meth)acrylate. The alicyclic bifunctional (meth)acrylate may be, for example, a compound having a structure represented by $R^1$-($L^1$)n1-Q-($L^2$)n2-$R^2$. Here, Q represents a divalent alicyclic group, $R^1$ and $R^2$ each independently represent a (meth)acryloyl group or a (meth)acryloyloxy group, $L^1$ and $L^2$ each independently represent a linking group, and n1 and n2 each independently represent 0 or 1. The divalent alicyclic group represented by Q is preferably an alicyclic hydrocarbon group having 3 to 20 carbon atoms, and examples thereof include a cyclopentylene group, a cyclohexylene group, a cycloheptylene group, a cyclooctylene group, a tricyclodecane group, and an adamantylene group. Examples of linking groups represented by $L^1$ and $L^2$ include an alkylene group. Examples of alkylene groups include an alkylene group having 1 to 6 carbon atoms.

Specific examples of alicyclic bifunctional (meth)acrylates include cyclohexanedimethanol di(meth)acrylate, ethoxylated cyclohexanedimethanol di(meth)acrylate, propoxylated cyclohexanedimethanol di(meth)acrylate, ethoxylated propoxylated cyclohexanedimethanol di(meth)acrylate, tricyclodecanedimethanol di(meth)acrylate, ethoxylated tricyclodecanedimethanol di(meth)acrylate, propoxylated tricyclodecanedimethanol di(meth)acrylate, and ethoxylated propoxylated tricyclodecanedimethanol di(meth)acrylate.

The component B containing one or more branch structures may be a bifunctional (meth)acrylate containing a branched alkylene group in one aspect. The number of carbon atoms of the branched alkylene group may be 1 or more, 2 or more, 3 or more or 4 or more. In addition, the number of carbon atoms of the branched alkylene group may be 10 or less, 9 or less, 8 or less, 7 or less, 6 or less or 5 or less. In one aspect, the branched alkylene group may include a quaternary carbon atom (that is, a carbon atom bonded to four carbon atoms). Specific examples of the component B containing one or more branch structures include neopentyl glycol di(meth)acrylate, ethoxylated neopentyl glycol di(meth)acrylate, and propoxylated neopentyl glycol di(meth)acrylate.

The molecular weight of the component B is not particularly limited, and may be, for example, in a range of 200 to 400 in one aspect. The component B may contain, as a (meth)acryloyl group, only an acryloyl group, only a methacryloyl group, or an acryloyl group and a methacryloyl group.

In the method of producing a polymerizable composition, when the component A and the component B are used as polymerizable compounds, the content of the component A based on a total amount (100 mass%) of (meth)acrylates used for producing the polymerizable composition is preferably 50.0 mass% or more, more preferably 60.0 mass% or more, and still more preferably 70.0 mass% or more. In addition, the content of the component A may be, for example, 95.0 mass% or less or 90.0 mass% or less. Only one type of component A may be used, and two or more types of component A may be used. When two or more types of component A are used, the above content is a total content of these two or more types of component A. This similarly applies to the content of other components such as the component B. Here, in the present invention and this specification, a component corresponding to both the component A and the component B may be regarded as the component A. On the other hand, the content of the component B based on a total amount (100 mass%) of (meth)acrylates used for producing the polymerizable composition may be 1.0 mass% or more, and is preferably 5.0 mass% or more and more preferably 10.0 mass% or more. In addition, the content of the component B may be, for example, 30.0 mass% or less, 25.0 mass% or less or 20.0 mass% or less. As the polymerizable compound used for producing the polymerizable composition, a (meth)acrylate other than the components A and B may be used or may not be used. When a (meth)acrylate other than the components A and B is used, the content thereof based on a total amount (100 mass%) of (meth)acrylates used for producing the polymerizable composition is preferably 10.0 mass% or less and more preferably 5.0 mass% or less. In addition, as the polymerizable compound used for producing the polymerizable composition, a polymerizable compound other than the (meth)acrylate may be used or may not be used. For example, the (meth)acrylate can be used in a proportion of 80.0 to 99.9 mass% based on a total amount (100 mass%) of all components used for producing the polymerizable composition. Here, the total amount of all components is, when a solvent is used, a total amount of all components excluding the solvent.

(Component C)

In the method of producing a polymerizable composition, as the polymerizable compound mixed with the above particles, a (meth)acrylate having a molecular weight of 400 or less and represented by the following Formula 2 may be exemplified.

[C3]

(Formula 2)

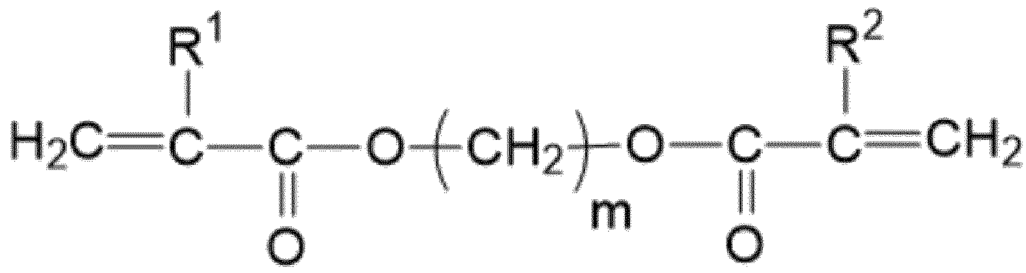

Such a (meth)acrylate is also called a "component C."

In Formula 2, $R^1$ and $R^2$ each independently represent a hydrogen atom or a methyl group, and m represents an integer of 1 or more. m is 1 or more, and may be, for example, 10 or less, 9 or less, 8 or less, 7 or less or 6 or less.

The molecular weight of the component C is 400 or less, and in order to further increase the coloring density of a photochromic layer formed from the polymerizable composition containing the component C and the photochromic compound, the molecular weight is preferably 350 or less, more preferably 300 or less and more preferably 250 or less. In addition, the molecular weight of the component C may be, for example, 100 or more, 150 or more or 200 or more.

The component C may contain, as a (meth)acryloyl group, only an acryloyl group, only a methacryloyl group, or an acryloyl group and a methacryloyl group. In one aspect, the component C preferably contains only an acryloyl group as a (meth)acryloyl group. Specific examples of the component C include 1,9-nonanediol diacrylate, 1,6-hexanediol diacrylate, and 1,10-decanediol diacrylate. According to studies performed by the inventors, in a photochromic layer formed from the polymerizable composition containing the component A and the component C as polymerizable compounds together with the photochromic compound, the photochromic compound that has received light to develop a color at a high density and the photochromic layer can exhibit excellent visible light transmission without light emission.

(Component D)

In the method of producing a polymerizable composition, as the polymerizable compound mixed with the above particles, an acyclic tri- or higher-functional (meth)acrylate may be exemplified. The component D can contribute to improving the performance such as coating suitability of the polymerizable composition and adhesion between the photochromic layer formed from the polymerizable composition and an adjacent layer. The component D is preferably a tri- to pentafunctional (meth)acrylate, more preferably a trifunctional or tetrafunctional (meth)acrylate, and still more preferably a trifunctional (meth)acrylate. Specific examples of the component D include pentaerythritol tri(meth)acrylate, pentaerythritol tetra(meth)acrylate, dipentaerythritol tetra(meth)acrylate, trimethylolethane tri(meth)acrylate, trimethylolpropane tri(meth)acrylate, ditrimethylolpropane tetra(meth)acrylate, tetramethylolmethane tetra(meth)acrylate, and tetramethylolmethane tri(meth)acrylate. The molecular weight of the component D may be, for example, in a range of 200 to 400, but is not limited to this range. The component D may contain, as a (meth)acryloyl group, only an acryloyl group, only a methacryloyl group, or an acryloyl group and a methacryloyl group. In one aspect, the acyclic tri- or higher-functional (meth)acrylate preferably contains, as a (meth)acryloyl group, only a methacryloyl group, that is, a methacrylate.

(Component E)

In the method of producing a polymerizable composition, as the polymerizable compound mixed with the above particles, a polymerizable compound having a viscosity of 100 cP (centipoises) or less and selected from the group consisting of (meth)acrylates and vinyl ether may be exemplified. Such a polymerizable compound is also referred to as a "component E." The component D can contribute to improving the performance such as coating suitability of the polymerizable composition and adhesion between the photochromic layer formed from the polymerizable composition and an adjacent layer. In the present invention and this specification, the "viscosity" is a value measured by a vibration type viscometer in the atmosphere at a temperature of 25°C. The viscosity of the component E is 100 cP or less, preferably 70 cP or less, and more preferably 50 cP or less. In addition, the viscosity of the component E may be, for example, 5 cP or more or 10 cP or more. The (meth)acrylate which is one aspect of the component E may be monofunctional to trifunctional, and is preferably monofunctional to bifunctional. In addition, the (meth)acrylate which is one aspect of the component E may contain an aryl group (for example, a phenyl group), an amide group and the like. In the present invention and this specification, the "vinyl ether" is a compound having one or more vinyl groups and one or more ether bonds in one molecule, and preferably has two or more vinyl groups in one molecule and more preferably has 2 to 4 vinyl groups. In addition, the number of ether bonds contained in the vinyl ether in one molecule is preferably 2 to 4. The molecular weight of the component E may be, for example, in a range of 150 to 250, but is not limited to this range. Specific examples of components E include 2-phenoxyethyl (meth)acrylate, acrylamide, methoxypolyethylene glycol (meth)acrylate, phenoxypolyethylene glycol (meth)acrylate, stearyl (meth)acrylate, 1,10-decanediol di(meth)acrylate, 1,6-hexanediol di(meth)acrylate, 1,9-nonanediol di(meth)acrylate, dipropylene glycol di(meth)acrylate, tripropylene glycol di(meth)acrylate, propylene glycol di(meth)acrylate, ethylene glycol di(meth)acrylate, diethylene glycol di(meth)acrylate, triethylene glycol di(meth)acrylate, tricyclodecanedimethanol di(meth)acrylate, ethoxylated propylene glycol di(meth)acrylate, trimethylolpropane tri(meth)acrylate, ethyl (meth)acrylate, butyl (meth)acrylate, isobutyl (meth)acrylate, tert-butyl (meth)acrylate, 2-ethylhexyl (meth)acrylate, isodecyl (meth)acrylate, dodecyl (meth)acrylate, tridecyl (meth)acrylate, diethylene glycol butyl ether (meth)acrylate, cyclohexyl (meth)acrylate, tetrahydrofurfuryl (meth)acrylate, benzyl (meth)acrylate, isobornyl (meth)acrylate, 2-(dimethylamino)ethyl (meth)acrylate, 2-(diethylamino)ethyl (meth)acrylate, glycidyl (meth)acrylate, polyethylene glycol di(meth)acrylate, tetramethylene glycol di(meth)acrylate, neopentyl glycol di(meth)acrylate, nonamethylene glycol di(meth)acrylate, isoamyl (meth)acrylate, ethylene glycol monovinyl ether, tetramethylene glycol mono vinyl ether, diethylene glycol mono vinyl ether, 2-ethylhexyl vinyl ether, 2-propenoic acid, 2-[2-(ethenyloxy)ethoxy]ethyl ester, and 2-(2-ethenoxyethoxy)ethyl 2-methylprop-2-enoate.

In the method of producing a polymerizable composition, when the component A and the component C are used as polymerizable compounds, the content of the component A based on a total amount of 100 mass% of the polymerizable compounds used for producing the polymerizable composition is preferably 50.0 mass% or more, more preferably 55.0 mass% or more, and still more preferably 60.0 mass% or more. In addition, the content of the component A may be 90.0 mass% or less, 85.0 mass% or less, 80.0 mass% or less or 75.0 mass% or less.

Regarding the component C, according to studies performed by the inventors, they observed that the coloring density of the photochromic compound when a color is developed under light emission in a photochromic layer formed from the polymerizable composition containing the component tends to be higher as the content of the component C is larger. On the other hand, according to studies performed by the inventors, they also observed that the fade rate of the photochromic compound after light emission tends to be slower as the content of the component C is larger. In consideration of the coloring density, the content of the component C based on a total amount of 100 mass% of the polymerizable compounds used for producing the polymerizable composition is preferably 5.0 mass% or more, more preferably 10.0 mass% or more and still more preferably 15.0 mass% or more. In addition, in order to prevent the fade rate from slowing down, the content of the component C is preferably 30.0 mass% or less and more preferably 25.0 mass% or less.

Regarding the component D, the content of the component D based on a total amount of 100 mass% of the polymerizable compounds used for producing the polymerizable composition may be 0 mass% and may be 0 mass% or more, more than 0 mass%, 1.0 mass% or more, 3.0 mass% or more, 5 mass% or more or 7 mass% or more. The content of the component D may be, for example, 20.0 mass% or less or 15.0 mass% or less. In one aspect, the component D, together with the component A and the component B, can be subjected to mixing with the above particles.

Regarding the component E, the content of the component E based on a total amount of 100 mass% of the polymerizable compounds used for producing the polymerizable composition may be 0 mass% and may be 0 mass% or more, more than 0 mass%, 1.0 mass% or more, 3.0 mass% or more, 5.0 mass% or more or 7.0 mass% or more. The content of the component E may be, for example, 20.0 mass% or less or 15.0 mass% or less. In one aspect, the component E, together with the component A and the component B, or additionally together with the component D, can be used for producing the polymerizable composition.

In the method of producing a polymerizable composition, the content of the polymerizable compound based on a total amount (100 mass%) of all components of the polymerizable composition may be, for example 80.0 mass% or more, 85.0 mass% or more or 90.0 mass% or more. Here, the total amount of all components is, when a solvent is used, a total amount of all components excluding the solvent. In addition, the content of the polymerizable compound may be, for example, 99.0 mass% or less, 95.0 mass% or less, 90.0 mass% or less or 85.0 mass% or less.

<Other Components>

As components of the polymerizable composition, in addition to the above particles and polymerizable compounds, one or more of various additives that can be generally added to the polymerizable composition, may be exemplified. One or more of these components can be used in an arbitrary proportion. As an example of such additives, a polymerization initiator that allows a polymerization reaction to proceed may be exemplified.

For example, regarding the polymerization initiator, a known polymerization initiator can be used, and a radical polymerization initiator is preferable, and it is more preferable that only a radical polymerization initiator be contained

as a polymerization initiator. In addition, regarding the polymerization initiator, a photopolymerization initiator or a thermal polymerization initiator can be used, and in order for a polymerization reaction to proceed in a short time, a photopolymerization initiator is preferable. Examples of photoradical polymerization initiators include benzoin ketals such as 2,2-dimethoxy-1,2-diphenylethane-1-one; α-hydroxyketones such as 1-hydroxycyclohexylphenyl ketone, 2-hydroxy-2-methyl-1-phenylpropan-1-one, and 1-[4-(2-hydroxyethoxy)phenyl]-2-hydroxy-2-methyl-1-propane-1-one; α-aminoketones such as 2-benzyl-2-dimethylamino-1-(4-morpholinophenyl)-butane-1-one, and 1,2-methyl-1-[4-(methylthio)phenyl]-2-morpholinopropan-1-one; oxime esters such as 1-[(4-phenylthio)phenyl]-1,2-octadione-2-(benzoyl)oxime; phosphine oxides such as bis(2,4,6-trimethylbenzoyl)phenylphosphine oxide, bis(2,6-dimethoxybenzoyl)-2,4,4-trimethylpentyl phosphine oxide, and 2,4,6-trimethylbenzoyldiphenylphosphine oxide; 2,4,5-triarylimidazole dimers such as 2-(o-chlorophenyl)-4,5-diphenylimidazole dimer, 2-(o-chlorophenyl)-4,5-di(methoxyphenyl)imidazole dimer, 2-(o-fluorophenyl)-4,5-diphenylimidazole dimer, 2-(o-methoxyphenyl)-4,5-diphenylimidazole dimer, and 2-(p-methoxyphenyl)-4,5-diphenylimidazole dimer; benzophenone compounds such as benzophenone, N,N'-tetramethyl-4,4'-diaminobenzophenone, N,N'-tetraethyl-4,4'-diaminobenzophenone, and 4-methoxy-4'-dimethylaminobenzophenone; quinone compounds such as 2-ethylanthraquinone, phenanthrenequinone, 2-tert-butylanthraquinone, octamethylanthraquinone, 1,2-benzanthraquinone, 2,3-benzanthraquinone, 2-phenylanthraquinone, 2,3-diphenylanthraquinone, 1-chloroanthraquinone, 2-methylanthraquinone, 1,4-naphthoquinone, 9,10-phenanthraquinone, 2-methyl-1,4-naphthoquinone, and 2,3-dimethylanthraquinone; benzoin ethers such as benzoin methyl ether, benzoin ethyl ether, and benzoin phenyl ether; benzoin compounds such as benzoin, methyl benzoin, and ethyl benzoin; benzyl compounds such as benzyl dimethyl ketal; acridine compounds such as 9-phenylacridine, 1,7-bis(9,9'-acridinyl heptane); and N-phenylglycine, and coumarin. In addition, in the 2,4,5-triarylimidazole dimer, substituents on the aryl groups of two triarylimidazole moieties may provide the same symmetric compound, or may provide different asymmetric compounds. In addition, a thioxanthone compound and a tertiary amine may be combined such as a combination of diethyl thioxanthone and dimethylaminobenzoic acid. Among these, in consideration of curability, transparency and heat resistance, α-hydroxyketone and phosphine oxide are preferable. The content of the polymerization initiator based on a total amount (100 mass%) of all components of the polymerizable composition may be, for example, in a range of 0.1 to 5.0 mass%. The total amount of all components is, when a solvent is used, a total amount of all components excluding the solvent.

As additives that can be used, known additives that can be generally added to the composition containing a photochromic compound, for example, additives such as a surfactant, an antioxidant, a radical scavenger, a light stabilizer, a UV absorbing agent, an anti-coloring agent, an antistatic agent, a fluorescent dye, a dye, a pigment, a fragrance, a plasticizer, and a silane coupling agent, may be exemplified. Known compounds can be used as these additives.

When the polymerizable composition is produced, various components described above can be added simultaneously or sequentially in any order and mixed.

The above particles and polymerizable compounds can be mixed with these components alone or with one or more other components in addition to these components. Examples of mixing methods include various mixing methods of mixing a plurality of components.

Examples of a preferable mixing method for further improving the solubility of the photochromic compound include mixing using an ultrasonic homogenizer. The ultrasonic homogenizer is a device that can generate bubbles in a liquid due to a pressure difference called cavitation according to ultrasonic vibration. It is speculated that, when the generated bubbles disappear, shock waves are generated, photochromic compound particles mixed with the polymerizable compound are crushed with the shock waves and made finer, and additionally, the polymerizable compound is heated with the energy of ultrasonic waves, which promotes molecule movements, and thus finely crushed photochromic compound particles are easily dispersed in the polymerizable compound. The inventors speculate that these factors contribute to further improving the solubility of the photochromic compound in the polymerizable compound (specifically, further reducing the dispersion particle size in the composition). However, the speculations described in this specification do not limit the present invention.

As the ultrasonic homogenizer, a commercially available ultrasonic homogenizer can be used, and examples of commercial products include THU-80 (commercially available from ASONE) and ultrasonic reaction device SR-500 HD-T (commercially available from Shinka Industry Co., Ltd.), but the present invention is not limited thereto. Mixing conditions for the ultrasonic homogenizer may be set according to the types of the photochromic compound and the polymerizable composition to be mixed, the concentrations of respective components in the composition and the like. For example, the output of the ultrasonic homogenizer may be 80 to 500 W, the frequency may be 20 to 80 kHz (preferably 30 to 80 kHz), and the ultrasonic application time (hereinafter also referred to as a "treatment time") of the ultrasonic homogenizer may be 5 to 240 minutes. However, in the method of producing a polymerizable composition, mixing conditions for the ultrasonic homogenizer are not limited to the above ranges.

During mixing using an ultrasonic homogenizer, in one aspect, the temperature of the polymerizable composition can be controlled by a temperature control unit. In addition, in another aspect, mixing using an ultrasonic homogenizer can be performed without temperature control by a temperature control unit. As described above, when mixing is performed using an ultrasonic homogenizer, the polymerizable composition is heated with the energy of ultrasonic waves.

Therefore, even if there is no temperature control by a temperature control unit, the temperature of the polymerizable composition during mixing can be made higher than before mixing. In order to further improve the solubility of the photochromic compound in the polymerizable compound, it is preferable to perform temperature control by a temperature control unit. Specifically, it is preferable to heat the polymerizable composition mixed using an ultrasonic homogenizer with a heating unit. As the heating unit, known heating units such as an oil bath, a water bath, and a heater can be used. Mixing using an ultrasonic homogenizer can be performed, for example, at a temperature of 80°C or higher, and is preferably performed at a temperature of 90°C or higher and more preferably at a temperature of 100°C or higher. Such a temperature may be, for example, 150°C or lower, 140°C or lower, 130°C or lower or 120°C or lower. The temperature described for mixing in the present invention and this specification is the highest temperature of the polymerizable composition during mixing. In one aspect, mixing using an ultrasonic homogenizer is preferably performed using an oil bath at a temperature of 100°C or higher. In addition, during mixing using an ultrasonic homogenizer, for example, a stirring unit such as a magnetic stirrer can be used in combination. When such a stirring unit is used in combination, liquid convection can occur in the polymerizable composition. This combination is preferable in order to uniformize the liquid temperature and uniformize a treatment using an ultrasonic homogenizer.

[Method of Producing Photochromic Article]

One aspect of the present invention relates to a method of producing a photochromic article including:

producing a polymerizable composition by the above production method; and
curing the produced polymerizable composition to form a cured product containing a photochromic compound.

In the present invention and this specification, the "photochromic article" is an article containing a photochromic compound. The photochromic article produced by the production method may be a photochromic article to which photochromism is imparted from an indeno-fused naphthopyran compound contained in the cured product of the polymerizable composition produced by the method of producing a polymerizable composition described above.

The cured product may be a substrate containing a photochromic compound in one aspect or may be a photochromic layer directly or indirectly provided on a substrate in another aspect.

The substrate containing a photochromic compound can be obtained by polymerizing and curing the polymerizable composition produced by the method of producing a polymerizable composition described above by a known method such as casting polymerization, and molding it into the shape of the substrate.

On the other hand, when a photochromic layer is directly or indirectly provided on a substrate, as the substrate, a substrate selected according to the type of the photochromic article may be used. The photochromic article may be an optical article in one aspect. Optical articles include various articles such as a spectacle lens, a goggle lens, a visor (cap) part of a sun visor, and a shield member of a helmet. As an example of the substrate, a spectacle lens substrate may be a plastic lens substrate or a glass lens substrate. The glass lens substrate may be, for example, a lens substrate made of inorganic glass. The lens substrate is preferably a plastic lens substrate because it is lightweight, hard to break, and easy to handle. Examples of plastic lens substrates include styrene resins such as (meth)acrylic resins, allyl carbonate resins such as polycarbonate resins, allyl resins, and diethylene glycol bis(allyl carbonate) resins (CR-39), vinyl resins, polyester resins, polyether resins, urethane resins obtained by reacting an isocyanate compound with a hydroxy compound such as diethylene glycol, thiourethane resins obtained by reacting an isocyanate compound with a polythiol compound, and a cured product (generally referred to as a transparent resin) obtained by curing a curable composition containing a (thio)epoxy compound having one or more disulfide bonds in the molecule. As the lens substrate, an undyed lens (colorless lens) may be used or a dyed lens (colored lens) may be used. The refractive index of the lens substrate may be, for example, about 1.60 to 1.75. However, the refractive index of the lens substrate is not limited to the above range, and may be within the above range, or may be above or below outside the above range. In the present invention and this specification, the refractive index is a refractive index for light having a wavelength of 500 nm. In addition, the lens substrate may be a lens having refractive power (so-called prescription lens) or a lens having no refractive power (so-called no-prescription lens). In order to clean the surface of the substrate or the like, one or more known pretreatments such as an alkaline treatment and a UV ozone treatment can be arbitrarily performed on the surface of the substrate before one or more layers are formed thereon.

The spectacle lens may include various lenses such as a single focus lens, a multifocal lens, and a progressive power lens. The type of the lens is determined by the surface shape of both sides of the lens substrate. In addition, the surface of the lens substrate may be a convex surface, a concave surface, or a flat surface. In a general lens substrate and spectacle lens, the object-side surface is a convex surface and the eyeball-side surface is a concave surface. However, the present invention is not limited thereto. The photochromic layer may be generally provided on the object-side surface of the lens substrate, or may be provided on the eyeball-side surface.

When the polymerizable composition produced by the method of producing a polymerizable composition described above is directly applied to the surface of the substrate or indirectly applied to the surface of the substrate with one or more other layers therebetween, and the applied composition is subjected to a curing treatment, a photochromic layer which is a cured layer obtained by curing the composition can be formed. Examples of other layers include a primer layer for improving the adhesion between the photochromic layer and the substrate. Such a primer layer is known. As the method of applying the polymerizable composition, known coating methods such as a spin coating method and a dip coating method can be used, and a spin coating method is preferable in consideration of coating uniformity. The curing treatment may be light emission and/or heat treatment, and light emission is preferable in order for the curing reaction to proceed in a short time. Curing treatment conditions may be determined according to the types of various components (polymerizable compounds, polymerization initiators and the like) contained in the polymerizable composition and the formulation of the polymerizable composition. The thickness of the photochromic layer formed in this manner is, for example, preferably in a range of 5 to 80 um, and more preferably in a range of 20 to 60 μm.

The photochromic article having the above photochromic layer may or may not have one or more functional layers in addition to the photochromic layer. Specific examples of functional layers include layers known as functional layers of optical articles such as a protective layer for improving durability, an anti-reflective layer, a water repellent or hydrophilic antifouling layer, and an anti-fogging layer.

One aspect of the photochromic article is a spectacle lens. In addition, as one aspect of the photochromic article, a goggle lens, a visor (cap) part of a sun visor, a shield member of a helmet and the like may be exemplified. These articles can exhibit an anti-glare function when they contain a photochromic compound.

In addition, since the spectacle lens is a photochromic article, in the eyeglasses including such a spectacle lens, for example, the photochromic compound contained in the spectacle lens develops a color when hit with sunlight outdoors, an anti-glare effect like that of sunglasses can be exhibited, and when returned to indoors, the photochromic compound can fade to restore transmission. Known techniques can be applied to the configuration of the frame and the like for the eyeglasses.

[Examples]

Hereinafter, the present invention will be described in more detail with reference to examples. However, the present invention is not limited to the embodiments shown in examples.

[Example 1 and Comparative Example 1]

For the indeno-fused naphthopyran compound having the same structure represented by Formula A described above, a method of adjusting the purity during synthesis, treatment conditions for solvent removal treatment during particle preparation and/or heat treatment conditions during particle preparation and the like were changed, and thus particles of Example 1 and Comparative Example 1 were obtained.

The degree of crystallization of respective particles was determined by the method described above.

Specifically, regarding the degree of crystallization, about 10 mg of the particle sample was filled in a Si nonreflective plate, X-ray diffraction analysis was performed using Spectris PANalytical Division X'Pert PROMPD as an X-ray diffractometer, the obtained results were analyzed by the Hermans method using analysis software bundled in the device, and the degree of crystallization was calculated by Formula 1 shown above. Measurement conditions for X-ray diffraction analysis were: target: Cu, X tube current/voltage: 40 mA/45 kV, and scanning range: $2\theta=4°$ to 65°. According to the X-ray diffraction analysis, it was confirmed that the particles of Example 1 were crystallographically amorphous particles, and the particles of Comparative Example 1 were particles in which crystallographically amorphous and crystalline particles were mixed.

In addition, the maximum primary particle size of respective particles was obtained by the method described above using a reflection type optical microscope (a magnification of 100).

[Evaluation of Solubility]

(Preparation of Polymerizable Composition)

In a glass container, 85 parts by mass of polyethylene glycol dimethacrylate (in Formula 1, n=14, R represents an ethylene group, a molecular weight of 736) and 15 parts by mass of tricyclodecane dimethanol dimethacrylate (a molecular weight of 332) were mixed to obtain a polymerizable compound mixture.

The particles of examples and comparative examples and the polymerization initiator were added to a polymerizable compound mixture obtained above and mixed using an ultrasonic homogenizer. As the polymerization initiator, a photoradical polymerization initiator (bis(2,4,6-trimethylbenzoyl)phenyl phosphine oxide (Omnirad 819 commercially available from IGM Resin B. V.)) was used. With respect to 100 parts by mass of the polymerizable compound mixture,

2.25 parts by mass of the above particles were added, and 0.8 parts by mass of the polymerization initiator was added. Mixing using an ultrasonic homogenizer was performed at a frequency of 20 kHz and an output of 80 W for a treatment time of 9 minutes. During mixing using an ultrasonic homogenizer, a magnetic stirrer was used in combination. Specifically, as the ultrasonic homogenizer, THU-80 (commercially available from ASONE) was used. THU-80 (commercially available from ASONE) is an ultrasonic homogenizer including an ultrasonic oscillator and a vibrator. For mixing, the container was placed on the magnetic stirrer, a vibrator was inserted into the mixture of the photochromic compound, the polymerizable compound and the polymerization initiator in the container, ultrasonic waves were oscillated from an ultrasonic oscillator, and the vibrator applied ultrasonic vibration to the mixture in the container. In this case, the mixture was stirred by rotating the rotor in the mixture in the container using a magnetic force of the magnetic stirrer. During mixing, an oil bath was used as a heating unit, and the mixture in the container was also heated (the highest temperature: 118°C).

Thereby, a polymerizable composition was prepared. 2 hours after the mixing was completed, a sample liquid for particle size distribution measurement was collected from the polymerizable composition while the liquid temperature was lowered, and the particle size distribution of the particles in the collected sample liquid was measured. As a particle size distribution meter, a zeta potential/particle size measurement system ELSZ-2 (commercially available from Otsuka Electronics Co., Ltd.) was used.

It can be evaluated that, when the dispersion particle size (average particle size) in the sample liquid measured by the particle size distribution meter was smaller, the solubility of the indeno-fused naphthopyran compound in the prepared polymerizable composition in the polymerizable compound was higher.

The dispersibility was evaluated based on the following evaluation criteria.

A: the dispersion particle size was 100 nm or less
B: the dispersion particle size was more than 100 nm

The above results are shown in Table 1.

[Table 1]

| | Degree of crystallization | Maximum primary particle size | Solubility |
|---|---|---|---|
| Example 1 | 5.30% | 180 μm | A |
| Comparative Example 1 | 7.18% | 850 μm | B |

Based on the results shown in Table 1, it was confirmed that the particles of Example 1 had excellent solubility in the polymerizable compound.

For example, the polymerizable composition prepared in Example 1 was applied onto a substrate and cured according to light emission, and thus a photochromic layer could be formed on the substrate.

Finally, the above aspects will be summarized.

According to one aspect, indeno-fused naphthopyran compound particles having a degree of crystallization of 10.00% or less and a maximum primary particle size of 200 um or less are provided.

According to the above particles, it is possible to produce a polymerizable composition in which an indeno-fused naphthopyran compound is dissolved in a polymerizable compound with high solubility.

In one aspect, the degree of crystallization of the particles may be 7.00% or less.

According to one aspect, there is provided a method of producing a photochromic article including producing a polymerizable composition by the above production method and curing the produced polymerizable composition to form a cured product containing a photochromic compound.

In one aspect, the cured product may be a photochromic layer, and the method of producing a photochromic article may include forming the photochromic layer on a substrate directly or indirectly.

In one aspect, the photochromic article may be a spectacle lens.

In one aspect, the photochromic article may be a goggle lens.

In one aspect, the photochromic article may be a visor part of a sun visor.

In one aspect, the photochromic article is a shield member of a helmet.

Two or more of the various aspects and forms described in this specification may be combined in arbitrary combinations.

The embodiments disclosed herein are only examples in all respects and should not be considered as restrictive. The scope of the present invention is not limited to the above description but is limited by the scope of claims, and is intended to encompass equivalents to and all modifications within the scope of the claims.

[Industrial Applicability]

The present invention is beneficial in the technical fields of eyeglasses, goggles, sun visors, helmets and the like.

## Claims

1. Indeno-fused naphthopyran compound particles having a degree of crystallization of 10.00% or less and a maximum primary particle size of 200 um or less.

2. The particles according to claim 1, which have a degree of crystallization of 7.00% or less.

3. A method of producing a polymerizable composition, comprising mixing the particles according to claim 1 or 2 with one or more polymerizable compounds.

4. A method of producing a photochromic article, comprising:

   producing a polymerizable composition by the production method according to claim 3; and
   curing the produced polymerizable composition to form a cured product containing a photochromic compound.

5. The method of producing a photochromic article according to claim 4, comprising

   forming a photochromic layer on a substrate directly or indirectly,
   wherein the cured product is the photochromic layer.

6. The method of producing a photochromic article according to claim 4 or 5, wherein the photochromic article is a spectacle lens.

7. The method of producing a photochromic article according to claim 4 or 5, wherein the photochromic article is a goggle lens.

8. The method of producing a photochromic article according to claim 4 or 5, wherein the photochromic article is a visor part of a sun visor.

9. The method of producing a photochromic article according to claim 4 or 5, wherein the photochromic article is a shield member of a helmet.

## INTERNATIONAL SEARCH REPORT

International application No. **PCT/JP2021/036066**

**A. CLASSIFICATION OF SUBJECT MATTER**

***C09K 9/02***(2006.01)i; ***G02C 7/10***(2006.01)i; ***C07D 311/78***(2006.01)i; ***C08F 2/44***(2006.01)i
FI: C07D311/78; C08F2/44 B; C09K9/02 B; G02C7/10

According to International Patent Classification (IPC) or to both national classification and IPC

**B. FIELDS SEARCHED**

Minimum documentation searched (classification system followed by classification symbols)

C09K9/02; G02C7/10; C07D311/78; C08F2/44

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| Y | JP 2014-507385 A (TRANSITIONS OPTICAL, INC.) 27 March 2014 (2014-03-27) claims, examples | 1-9 |
| Y | JP 2014-502604 A (TRANSITIONS OPTICAL, INC.) 03 February 2014 (2014-02-03) claims, examples | 1-9 |
| Y | JP 2009-526092 A (TRANSITIONS OPTICAL, INC.) 16 July 2009 (2009-07-16) claims, examples | 1-9 |
| Y | WO 2011/034202 A1 (TOKUYAMA CORP.) 24 March 2011 (2011-03-24) page 4, lines 8-11, examples | 1-9 |
| Y | JP 2004-514774 A (TRANSITIONS OPTICAL, INC.) 20 May 2004 (2004-05-20) paragraph [0004] | 1-9 |
| Y | JP 2003-519694 A (PPG INDUSTRIES OHIO, INC.) 24 June 2003 (2003-06-24) paragraphs [0008], [0009] | 1-9 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **10 November 2021** | **22 November 2021** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**
**Information on patent family members**

International application No.
**PCT/JP2021/036066**

| Patent document cited in search report | Publication date (day/month/year) | Patent family member(s) | Publication date (day/month/year) |
|---|---|---|---|
| JP 2014-507385 A | 27 March 2014 | US 2012/0156521 A1<br>claims, examples<br>WO 2012/082506 A1<br>EP 2651912 A1<br>CN 103354810 A<br>KR 10-2014-0014107 A | |
| JP 2014-502604 A | 03 February 2014 | US 2011/0129678 A1<br>claims, examples<br>WO 2012/082299 A1<br>EP 2652552 A1<br>CN 103339564 A<br>KR 10-2013-0100795 A | |
| JP 2009-526092 A | 16 July 2009 | US 2007/0138448 A1<br>claims, examples<br>WO 2007/078529 A1<br>EP 1966180 A1<br>KR 10-2008-0078671 A<br>CN 101341141 A | |
| WO 2011/034202 A1 | 24 March 2011 | US 2012/0170098 A1<br>paragraph [0013], examples<br>EP 2479171 A1<br>CN 102471304 A<br>KR 10-2012-0053988 A | |
| JP 2004-514774 A | 20 May 2004 | US 2003/0045612 A1<br>paragraph [0005]<br>WO 2002/044258 A2<br>EP 1340108 A2 | |
| JP 2003-519694 A | 24 June 2003 | US 2002/0007078 A1<br>paragraphs [0008], [0009]<br>WO 2001/051483 A1<br>EP 1248778 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5645767 A